# EUROPEAN PATENT SPECIFICATION

(11) **EP 2 497 434 B1**
(45) Date of publication and mention of the grant of the patent: **15.10.2014**
(21) Application number: 12158575.6
(22) Date of filing: 08.03.2012
(51) Int. Cl.: A61B 17/34, A61N 1/05

(54) **Epidural needle for spinal cord stimulation**
Epiduralnadel zur Rückenmarkstimulation
Aiguille péridurale de stimulation de la moelle épinière

(30) Priority: 11.03.2011 US 201113046282
(43) Date of publication of application: 12.09.2012
(73) Proprietor: Greatbatch Ltd., Clarence, NY 14031 (US)
(72) Inventor: Daglow, Terry Douglas, Bonham, Texas 75418 (US); Geroy, Jesse, Ham Lake, Minnesota 55304 (US)
(74) Representative: Lecomte & Partners

(56) References cited:
- EP-A1- 2 226 022
- US-A- 5 255 691
- US-A- 5 389 079
- US-A1- 2004 210 245

## Description

### Field

The present disclosure relates to an epidural needle with three or more components for implanting therapy delivery elements, such as for example, stimulation leads in the epidural space of a patient. The inner cannula substantially extends across the elongated opening at the distal end of the outer cannula to form a seal with the ligament during "loss of resistance" testing. Loss of resistance testing is performed by removing the stylet from the inner cannula. Once the epidural needle is positioned in the epidural space, the inner cannula is removed to facilitate implantation of a therapy delivery element in the epidural space.

### Background

Implantable neurostimulation systems have proven therapeutic in a wide variety of diseases and disorders. Pacemakers and Implantable Cardiac Defibrillators (ICDs) have proven highly effective in the treatment of a number of cardiac conditions (e.g., arrhythmias). Spinal Cord Stimulation (SCS) systems have long been accepted as a therapeutic modality for the treatment of chronic pain syndromes, and the application of tissue stimulation has begun to expand to additional applications such as angina pectoralis and incontinence. Deep Brain Stimulation (DBS) has also been applied therapeutically for well over a decade for the treatment of refractory chronic pain syndromes, and DBS has also recently been applied in additional areas such as movement disorders and epilepsy. Peripheral Nerve Stimulation (PNS) systems have demonstrated efficacy in the treatment of chronic pain syndromes and incontinence, and a number of additional applications are currently under investigation. Functional Electrical Stimulation (FES) systems such as the Freehand system by NeuroControl (Cleveland, Ohio) have been applied to restore some functionality to paralyzed extremities in spinal cord injury patients.

Each of these implantable neurostimulation systems typically includes one or more therapy delivery elements implanted at the desired stimulation site and an implantable neurostimulator, such as an implantable pulse generator (IPG), implanted remotely from the stimulation site, but coupled either directly to the therapy delivery elements or indirectly to the therapy delivery elements via one or more extensions in cases where the length of the therapy delivery elements is insufficient to reach the IPG. In some cases, the extension leads may be used to facilitate coupling of the neurostimulator, which may otherwise be incompatible with the therapy delivery elements or extension leads, thereto. Thus, electrical pulses can be delivered from the neurostimulator to the therapy delivery elements to stimulate the tissue and provide the desired efficacious therapy to the patient.

In the context of an SCS procedure, one or more therapy delivery elements are introduced through the patient's back into the epidural space under fluoroscopy, such that the electrodes carried by the leads are arranged in a desired pattern and spacing to create an electrode array. The specific procedure used to implant the therapy delivery elements will ultimately depend on the type of therapy delivery elements used. Currently, there are two types of commercially available therapy delivery elements: a percutaneous lead and a surgical lead.

A percutaneous lead includes a cylindrical body with ring electrodes, and can be introduced into contact with the affected spinal tissue through a Touhy-like needle, which passes through the skin, between the desired vertebrae, and into the epidural space above the dura layer. For unilateral pain, a percutaneous lead is placed on the corresponding lateral side of the spinal cord. For bilateral pain, a percutaneous lead is placed down the midline of the spinal cord, or two percutaneous leads are placed down the respective sides of the midline. In many cases, a stylet, such as a metallic wire, is inserted into a lumen running through the center of each of the percutaneous leads to aid in insertion of the lead through the needle and into the epidural space. The stylet gives the lead rigidity during positioning, and once the lead is positioned, the stylet can be removed after which the lead becomes flaccid.

U.S. Pat. No. 6,554,809 discloses a needle including a hollow shaft having opposed distal and proximal ends, the hollow shaft having a lumen extending from the proximal end of the shaft and terminating at an opening on a top of and proximal to the distal end of the needle shaft. A cutting surface is at the distal end of the hollow shaft is adapted to be inserted into a patient, wherein the cutting surface is on the bottom of the distal end of the hollow shaft.

U.S. Pat. No. 4,141,365 discloses a tissue stimulation apparatus for positioning a lead adjacent to tissue that is to be stimulated electrically. The apparatus particularly includes a body penetration and insertion assembly that carries the electrode into contacting relation with the tissue. The insertion assembly includes a hollow needle having a slot formed longitudinally along the length of one wall thereof. The slot allows transverse removal of the flexible lead from the needle after proper positioning of the lead and after removal of the needle from the body. The slotted assembly allows the flexible electrode lead to pass through the hollow needle.

U.S. Pat. No. 5,255,691 discloses an epidural needle assembly, including an elongated needle having a side opening at its distal tip, a hub at its proximal tip and a lumen extending there between. A removable stylet with a beveled tip is inserted into the lumen of the needle through the hub. The distal tip may be curved in the direction of the side opening when unrestrained by the stylet.

U.S. Pat. No. 6,104,960 discloses a system and method for providing medical electrical stimulation to a portion of the nervous system. The system includes a rigid hollow needle having a lumen and a flexible lead body disposed within the lumen of the needle. The lead body has an insulated coiled proximal section and an electrode section.

US 2004/210245 A1 discloses an epidural needle for implanting therapy delivery elements in an epidural space, the epidural needle comprising: an outer cannula comprising an outer hub at a proximal end, an elongated opening at a curved distal end, and a lumen extending between the outer hub and the elongated opening, the lumen of the outer cannula sized to permit passage of the therapy delivery elements; and a stylet comprising a stylet hub, a distal end, and an outer diameter sized to slide freely within the lumen of the outer cannula, the distal end of the stylet extending substantially across the opening of the outer cannula.

One potential risk involving placement of leads in the epidural space is the accidental puncture of the dura, and damage to the spinal cord. Identification of the precise moment when the needle is advanced into the epidural space decreases the likelihood of that risk.

One method for identifying this space is the "loss of resistance" techniques. The loss of resistance technique involves direction of the epidural needle through the skin into the interspinous ligament. The stylet in the needle is removed and an air-tight and free sliding glass syringe, containing air, or saline is connected to the needle. If the needle tip is positioned within the substance of the interspinous ligament, injection will not be possible. Proper positioning of the needle is defined as the feeling of resistance. At this point, most textbooks suggest for the non-injecting hand to advance the needle with the thumb and index finger grasping the hub of the needle while the dorsum of the hand rests on the patient's back for stabilization. The injecting hand is placed on the plunger of the syringe with gentle but continuous pressure. As the needle passes through the ligarnentum flavum and enters the epidural space, a sudden loss of resistance occurs.

There are several disadvantages to this technique. First, the method described above is especially difficult for a novice because experience is required to obtain coordination of the two hands which are functioning differently. Second, because of the lack of an objective visual indicator, this method is difficult to supervise and results in a high incidence of dural puncture among novices.

The "loss of resistance" technique has widely been alternated involving a two-handed grip on the syringe and needle with continuous firm pressure on the hub. As the needle is advanced a few millimeters ("mm"), the surgeon will stop and check the location of the needle by gently depressing the plunger and confirming whether the needle tip is still within the ligament or has moved to the area where loss of resistance occurs. The apparent disadvantage of this method is that in between stops, the needle could have advanced through the epidural space and punctured the dura.

A variation of the loss of resistance technique is referred to as the "hanging drop" technique. The "hanging drop" technique capitalizes upon the loss of pressure experienced when the needle enters the epidural space. A drop of saline solution is placed on the open hub of the needle. The drop "hangs" on the needle until the needle enters the epidural space, when the needle tip indents the dura resulting in negative pressure and the drop is "sucked" into the needle from the change in pressure. This indicates that the needle should be stopped as it has entered the epidural space.

Regardless of the technique used, locating the epidural space can be a difficult endeavor for both novices and experts because it is a potential space between two tissues held together by a slight negative pressure. Dural puncture is the greatest risk when there is error, with consequences ranging from spinal headaches to spinal cord damage.

### Brief Summary

The present invention is directed to an epidural needle for implanting therapy delivery elements in an epidural space. The epidural needle includes at least an outer cannula containing an inner cannula, and a stylet located within the lumen of the inner cannula. The inner cannula substantially extends across the elongated opening at the distal end of the outer cannula to form a seal with the ligament during "loss of resistance" testing. Loss of resistance testing is performed by removing the stylet from the inner cannula. Once the epidural needle is positioned in the epidural space, the inner cannula is removed to facilitate implantation of a therapy delivery element in the epidural space.

The outer cannula includes an outer hub at a proximal end that engages with inner hub on the inner cannula to fix the rotational orientation of the inner cannula relative to the outer cannula.

The distal end of the stylet is configured to extend substantially across the opening of the inner cannula. In one embodiment, engagement features are provided on the stylet hub and the inner hub to maintain the inner cannula in a fixed rotational orientation relative to the stylet when the stylet. The outer cannula, the inner cannula and the stylet are preferably made from a malleable material to facilitate shaping by the surgeon.

The elongated opening in the outer cannula includes an angled distal end with a perimeter edge, and a portion of a sidewall of the outer cannula removed. The inner cannula substantially extends across removed portion of the sidewall of the outer cannula to create a seal with the ligament during loss of resistance testing. The perimeter edge of the opening in the inner cannula substantially extends along perimeter edge of the angled distal end of the outer cannula. The elongated opening preferably has a length of about 8 mm to about 12 mm and the opening at the distal end of the inner cannula has a length of about 2 mm to about 3 mm.

A neurostimulation system including an implantable pulse generator and a therapy delivery element is also disclosed. A proximal end of the therapy delivery element is adapted to electrically couple with the implantable pulse generator and a distal end includes a plurality of electrodes electrically coupled to the implantable pulse generator. An epidural needle in accordance with an embodiment of the present disclosure is provided for implanting the therapy delivery elements in an epidural space

A method of implanting therapy delivery elements in an epidural space through an epidural needle is also disclosed. An inner cannula is positioned in a lumen of an outer cannula so an elongated distal opening in the outer cannula is substantially covered by the inner cannula. A stylet is positioned in the lumen of inner cannula so an opening in distal end of the inner cannula is covered. The distal end of the epidural needle is advanced into the living body toward the epidural space. The stylet is removed from the inner cannula to perform the loss of resistance test. Pressurized air is delivered to the lumen of the inner cannula. A reduction in pressure of the pressurized air is sensed when the distal end of the inner cannula needle enters the epidural space. The inner cannula is then removed from the outer cannula. Distal end of the therapy delivery element is inserted through the outer cannula and into the epidural space. The outer cannula is removed while leaving the therapy delivery element in the epidural space. Proximal end of the therapy delivery element is electrically coupled to an implantable pulse generator.

An upper edge of opening of the inner cannula may for instance be located within about 3 mm to about 4 mm from the distal end of the outer cannula. Moreover, at least one of the inner cannula and the stylet may comprise a polymeric material or a super elastic metal.

### Brief Description of the Several Views of the Drawing

Figure 1 is a schematic illustration of a therapy delivery system.
Figure 2 is a schematic illustration of an environment for a therapy delivery system in accordance with an embodiment of the present disclosure.
Figure 3 is an alternate illustration of the environment for an implantable pulse generator with a therapy delivery element in accordance with an embodiment of the present disclosure.
Figure 4 is a schematic illustrate on a conventional epidural needle used to implant therapy delivery elements to an epidural space.
Figure 5 is a schematic illustration of an epidural needle located in an epidural space.
Figures 6A and 6B are perspective views of an outer cannula for an epidural needle in accordance with an embodiment of the present disclosure.
Figures 7A and 7B are perspective views of an inner cannula for an epidural needle in accordance with an embodiment of the present disclosure.
Figures 8A and 8B are perspective views of a stylet for an epidural needle in accordance with an embodiment of the present disclosure.
Figure 9 is a schematic illustration of an epidural needle in accordance with an embodiment of the present disclosure located in an epidural space.
Figure 10 is a flow diagram of a method of implanting therapy delivery elements in an epidural space using an epidural needle in accordance with an embodiment of the present disclosure.

### Detailed Description of Some Embodiments

The description that follows relates to spinal cord stimulation (SCS) system.

However, it is to be understood that the while the present disclosure lends itself well to applications in SCS, the disclosure in its broadest aspects may not be so limited. Rather, the disclosure may be used with any type of implantable therapy delivery system with one or more therapy delivery elements. For example, the present disclosure may be used as part of a pacemaker, a defibrillator, a cochlear stimulator, a retinal stimulator, a stimulator configured to produce coordinated limb movement, a cortical stimulator, a deep brain stimulator, peripheral nerve stimulator, microstimulator, or in any other neural stimulator configured to treat urinary incontinence, sleep apnea, shoulder sublaxation, headache, etc.

In another embodiment, one or more of the therapy delivery elements may be a fluid delivery conduit, such as a catheter, including an inner lumen that is placed to deliver a fluid, such as pharmaceutical agents, insulin, pain relieving agents, gene therapy agents, or the like from a fluid delivery device (e.g., a fluid reservoir and/or pump) to a respective target tissue site in a patient.

In yet another embodiment, one or more of the therapy delivery elements may be an electrical lead including one or more sensing electrodes to sense physiological parameters (e.g., blood pressure, temperature, cardiac activity, etc.) at a target tissue site within a patient. In the various embodiments contemplated by this disclosure, therapy may include stimulation therapy, sensing or monitoring of one or more physiological parameters, fluid delivery, and the like. "Therapy delivery element" includes pacing or defibrillation leads, stimulation leads, sensing leads, fluid delivery conduit, and any combination thereof. "Target tissue site" refers generally to the target site for implantation of a therapy delivery element, regardless of the type of therapy.

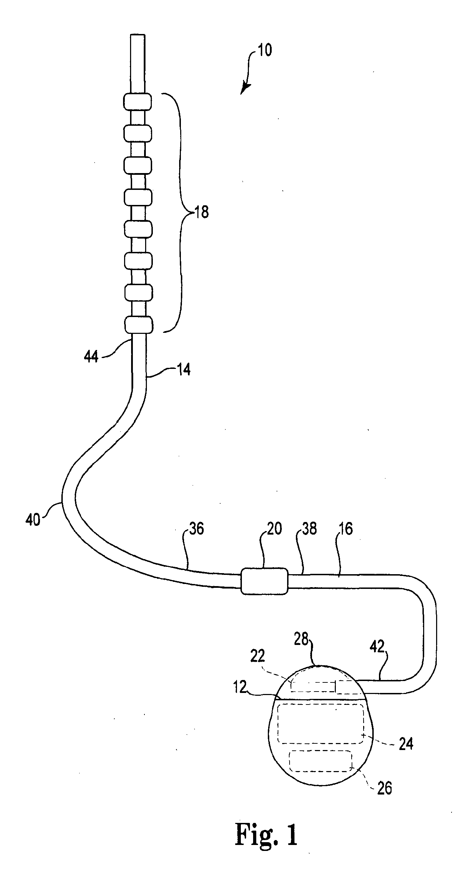

Figures 1 illustrates a generalized therapy delivery system 10 that may be used in spinal cord stimulation (SCS), as well as other stimulation applications. The therapy delivery system 10 generally includes an implantable pulse generator 12, an implantable therapy delivery element 14, which carries an array of electrodes 18 (shown exaggerated for purposes of illustration), and an optional implantable extension lead 16. Although only one therapy delivery element 14 is shown, typically two or more therapy delivery elements 14 are used with the therapy delivery system 10.

The therapy delivery element 14 includes elongated body 40 having a proximal end 36 and a distal end 44. The elongated body 40 typically has a diameter of between about 0.03 inches to 0.07 inches and a length within the range of 30 cm to 90 cm for spinal cord stimulation applications. The elongated body 40 may be composed of a suitable electrically insulative material, such as, a polymer (e.g., polyurethane or silicone), and may be extruded from as a uni-body construction.

In the illustrated embodiment, proximal end 36 of the therapy delivery element 14 is electrically coupled to distal end 38 of the extension lead 16 via a connector 20, typically associated with the extension lead 16. Proximal end 42 of the extension lead 16 is electrically coupled to the implantable pulse generator 12 via connector 22 associated with housing 28. Alternatively, the proximal end 36 of the therapy delivery element 14 can be electrically coupled directly to the connector 20.

In the illustrated embodiment, the implantable pulse generator 12 includes electronic subassembly 24 (shown schematically), which includes control and pulse generation circuitry (not shown) for delivering electrical stimulation energy to the electrodes 18 of the therapy delivery element 14 in a controlled manner, and a power supply, such as battery 26.

The implantable pulse generator 12 provides a programmable stimulation signal (e.g., in the form of electrical pulses or substantially continuous-time signals) that is delivered to target stimulation sites by electrodes 18. In applications with more than one therapy delivery element 14, the implantable pulse generator 12 may provide the same or a different signal to the electrodes 18.

Alternatively, the implantable pulse generator 12 can take the form of an implantable receiver-stimulator in which the power source for powering the implanted receiver, as well as control circuitry to command the receiver-stimulator, are contained in an external controller inductively coupled to the receiver-stimulator via an electromagnetic link. In another embodiment, the implantable pulse generator 12 can take the form of an external trial stimulator (ETS), which has similar pulse generation circuitry as an IPG, but differs in that it is a non-implantable device that is used on a trial basis after the therapy delivery element 14 has been implanted and prior to implantation of the IPG, to test the responsiveness of the stimulation that is to be provided.

The housing 28 is composed of a biocompatible material, such as for example titanium, and forms a hermetically sealed compartment containing the electronic subassembly 24 and battery 26 are protected from the body tissue and fluids. The connector 22 is disposed in a portion of the housing 28 that is, at least initially, not sealed. The connector 22 carries a plurality of contacts that electrically couple with respective terminals at proximal ends of the therapy delivery element 14 or extension lead 16. Electrical conductors extend from the connector 22 and connect to the electronic subassembly 24.

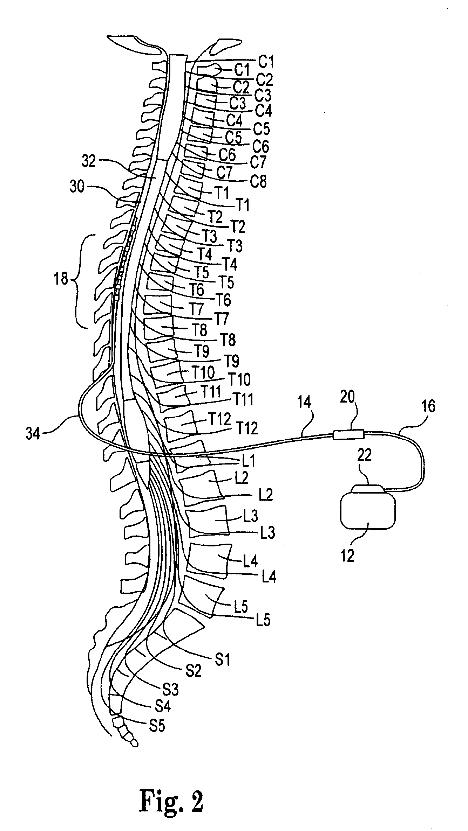

Figure 2 is a side skeletal view of a human body illustrating spinal column. The sacrum region is at a lower end of the spinal column below L-5 and adjacent the pelvic region. The sacrum is a triangular-shaped bone formed generally by five fused vertebrae, i.e., sacral vertebrae that are wedged dorsally between the two hip bones of the pelvic region in this region of the human anatomy. The lumbar region extends from L-1 to L-5 between the sacrum region at a lower end and the thorax region (T-1 to T-12) at an upper end. The thorax region extends from T-12 to T-1 at the base of the cervical region. The cervical region extends from C1 to C7.

The therapy delivery element 14 is implanted in the epidural space 30 of a patient in close proximity to the dura, the outer layer that surrounds the spinal cord 32, to deliver the intended therapeutic effects of spinal cord electrical stimulation. The target stimulation sites may be anywhere along the spinal cord 32, such as for example proximate the sacral nerves.

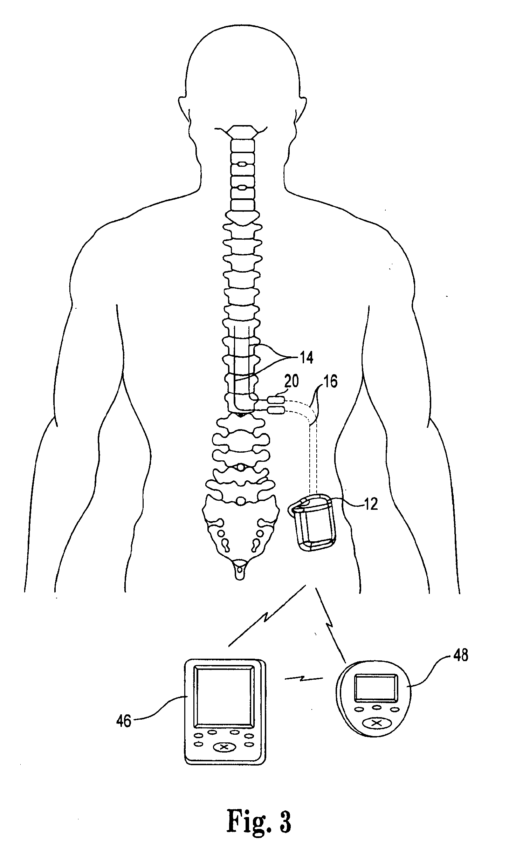

Because of the lack of space near the lead exit point 34 where the therapy delivery element 14 exits the spinal column, the implantable pulse generator 12 is generally implanted in a surgically-made pocket either in the abdomen or above the buttocks, such as illustrated in Figure 3. The implantable pulse generator 12 may, of course, also be implanted in other locations of the patient's body. Use of the extension lead 16 facilitates locating the implantable pulse generator 12 away from the lead exit point 34. In some embodiments, the extension lead 16 serves as a lead adapter if the proximal end 36 of the therapy delivery element 14 is not compatible with the connector 22 of the implantable pulse generator 12, since different manufacturers use different connectors at the ends of their stimulation leads and are not always compatible with the connector 22.

As illustrated in Figure 3, the therapy delivery system 10 also may include a clinician programmer 46 and a patient programmer 48. Clinician programmer 46 may be a handheld computing device that permits a clinician to program neurostimulation therapy for patient using input keys and a display. For example, using clinician programmer 46, the clinician may specify neurostimulation parameters for use in delivery of neurostimulation therapy. Clinician programmer 46 supports telemetry (e.g., radio frequency telemetry) with the implantable pulse generator 12 to download neurostimulation parameters and, optionally, upload operational or physiological data stored by implantable pulse generator 12. In this manner, the clinician may periodically interrogate the implantable pulse generator 12 to evaluate efficacy and, if necessary, modify the stimulation parameters.

Similar to clinician programmer 46, patient programmer 48 may be a handheld computing device. Patient programmer 48 may also include a display and input keys to allow patient to interact with patient programmer 48 and the implantable pulse generator 12. The patient programmer 48 provides patient with an interface for control of neurostimulation therapy provided by the implantable pulse generator 12. For example, patient may use patient programmer 48 to start, stop or adjust neurostimulation therapy. In particular, patient programmer 48 may permit patient to adjust stimulation parameters such as duration, amplitude, pulse width and pulse rate, within an adjustment range specified by the clinician via clinician programmer 48, or select from a library of stored stimulation therapy programs.

The implantable pulse generator 12, clinician programmer 46, and patient programmer 48 may communicate via cables or a wireless communication. Clinician programmer 46 and patient programmer 48 may, for example, communicate via wireless communication with the implantable pulse generator 12 using RF telemetry techniques known in the art. Clinician programmer 46 and patient programmer 48 also may communicate with each other using any of a variety of local wireless communication techniques, such as RF communication according to the 802.11 or Bluetooth specification sets, infrared communication, e.g., according to the IrDA standard, or other standard or proprietary telemetry protocols.

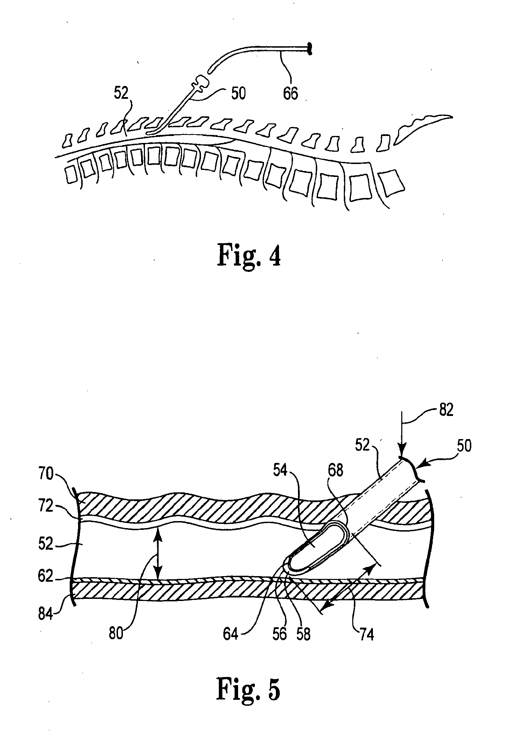

Figures 4 and 5 illustrates a conventional Touhy needle 50 located in epidural space 52. The illustrated needle 50 includes outer cannula 52 and inner cannula 54. Cutting edge 56 is located at distal end 58 of the outer cannula 52. The cutting edge 56 is typically located at about the level of centerline of the outer cannula 52, making it less likely to penetrate the dura 62. Removal of the inner cannula 54 exposes opening 64 at distal end 56. The opening 64 is configured to permit therapy delivery elements 66 to be advanced into, and withdrawn from, the epidural space 52.

Upper edge 68 of the opening 64 is typically set back a distance 74 of about 7 mm to about 9 mm from the distal end 56 to reduce the chance that the therapy delivery element 66 will catch on the needle 50 during withdrawal. In the illustrated embodiment, the upper edge 68 remains in the ligament 72, thereby minimizing interference of the upper edge 68 with removal of the therapy delivery element 66.

In order to use the loss of resistance technique to determine the location of the needle 50 in the epidural space 52, the upper edge 68 of the opening 64 must be sealed against the ligament 72. As noted above, to perform the loss of resistance technique, the inner cannula 54 is removed and the surgeon applies air pressure through the outer cannula 52. Due to the length 74 of the opening 64, however, the distal end 54 of the needle 50 may already have punctured the dura 62 by the time the upper edge 68 of the opening 64 is positioned in the yellow ligament 72.

Alternatively, if the upper edge 68 of the opening 64 is still located in the muscle tissue 70 rather than the ligament 72, air pressure delivered through the outer cannula 52 may leak into the muscle tissue 70, creating a false indication that the distal end 58 is properly located in the epidural space 52, making it difficult to use the loss of resistance technique.

A typical Touhy needle 50 used to implant therapy delivery elements between the L1 and T12 vertebrae requires an epidural space 80 of up to about 4 mm to function properly. In some embodiments, the surgeon can apply a downward pressure 82 on the dura 62 using the distal end 58 of the needle 50 in order to increase the space 80 by compressing the spinal cord 84. As the spinal cord 84 branches off into smaller nerve bundles, however, little or no spinal cord compression is possible. Also, outside of the L1 to T12 region the epidural space is smaller and more difficult to access safely.

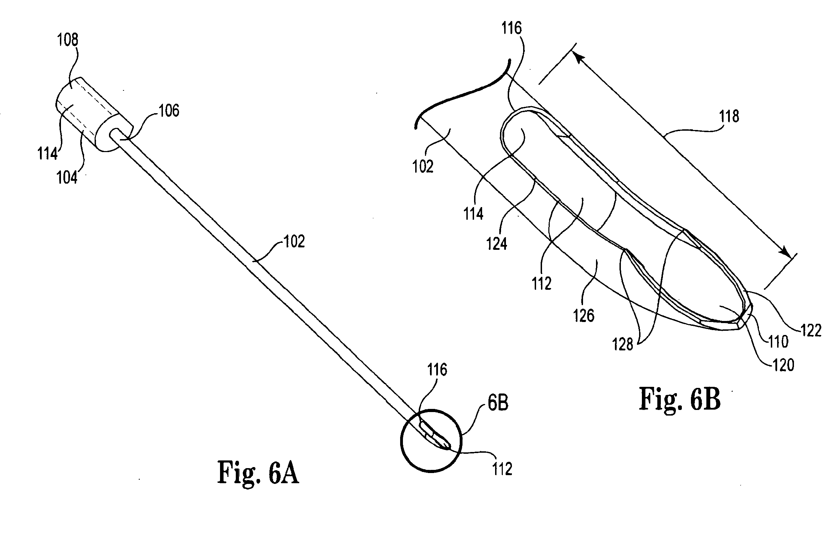

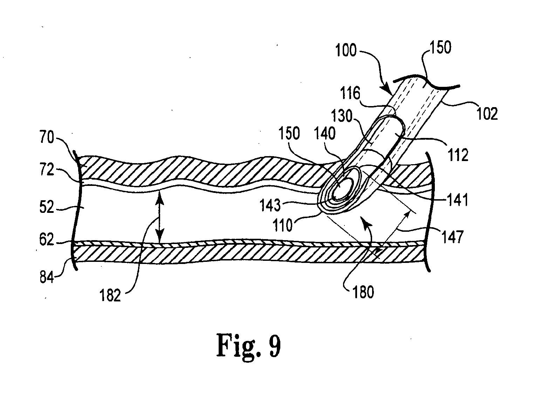

Figures 6 through 9 illustrate an epidural needle 100 with outer cannula 102, an inner cannula 130, and a stylet 150, in accordance with an embodiment of the present disclosure. Figures 6A and 6B illustrate the outer cannula 102 of the epidural needle 100 illustrated in Figure 9. The outer cannula 102 includes outer hub 104 at proximal end 106 with non-circular receiving opening 108.

Lumen 114 extends from the recess 108 in the hub 104 to the elongated opening 112. Distal end 110 includes elongated opening 112 configured to permit a therapy delivery element 14 to be delivered into, and removed from, an epidural space 30, without interference from upper edge 116.

In the illustrated embodiment, the elongated opening 112 includes an angled distal end 110 of the outer cannula 102. The angled distal end 110 includes perimeter edge 122. Portion 124 of the sidewall 126 of the outer cannula 102 is removed to form part of the opening 112. In the illustrated embodiment, the portion 124 of the sidewall 126 intersects perimeter edge 122 at transition locations 128. The elongated opening 112 preferably has a length 118 of about 8 mm to about 12 mm to reduce the chance that the therapy delivery element 14 will catch on the needle 100 during withdrawal. The outer cannula 102 is preferably constructed from a bendable or ductile material that can be pre-shaped by the surgeon.

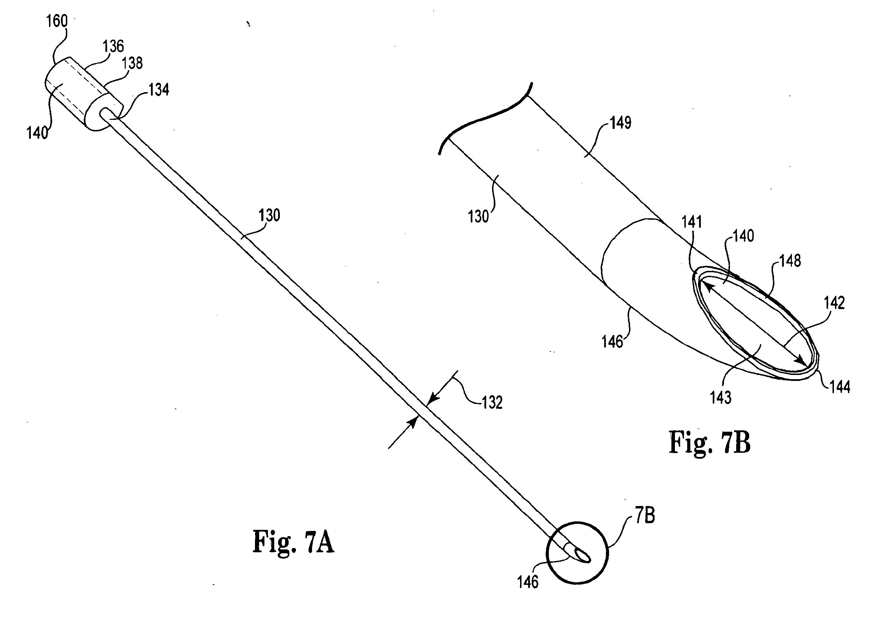

Figures 7A and 7B illustrate inner cannula 130 of the epidural needle 100 illustrated in Figure 9. The inner cannula 130 has an outside diameter 132 sized to slide within lumen 114. Lumen 140 extends from hub 136 to opening 143 at distal end 144 of the inner cannula 130. The opening 143 preferably has a length 142 from upper edge 141 to distal end 144 of about 2 mm to about 3 mm. Although the inner cannula 130 is illustrated as a one-piece structure, it is possible for it to be constructed as a multi-component assembly.

Proximal end 134 includes inner hub 136 with a non-circular surface 138 is configured to engage with receiving opening 108 in the outer hub 104. Engagement of the inner hub 136 in the receiving opening 108 of the outer hub 104 results in the opening 143 of the inner cannula 130 oriented in generally the same direction as the opening 112 of the outer cannula 102. Curvature 146 at the distal end 144 is preferably shaped to reside in curved recess 120 so that perimeter 148 of the opening 143 is positioned against perimeter 122 of at distal end 110 of the outer cannula 102.

As best illustrated in Figure 9, shaft portion 149 of the inner cannula 130 substantially fills the balance of the elongated opening 112 in the outer cannula 102. The distal end 144 of the inner cannula 130 substantially fills the elongated opening 112 of the outer cannula 102. In the preferred embodiment, the inner cannula 130 fills more than about 50%, and more preferably about 75% of the elongated opening 112, so that the opening 143 is located at the most distal end 110 of the outer cannula 102.

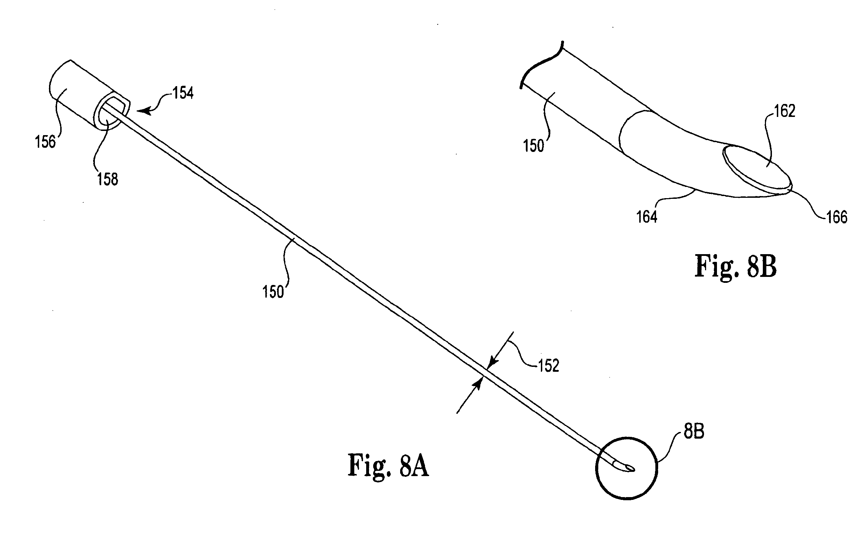

Figures 8A and 8B illustrate stylet 150 of the epidural needle 100 of Figure 9. The stylet 150 has an outside diameter 152 sized to slide within lumen 140 of the inner cannula 130. Proximal end 154 of the stylet 150 includes stylet hub 156 with non-circular receiving opening 158 sized to engage with proximal end 160 of inner hub 136. Although the stylet 150 is illustrated as a one-piece structure, it is possible for the stylet 150 to be constructed as a multi-component assembly. The hubs 136, 156 orient the stylet 150 so flat surface 162 at distal end 164 extends across opening 143 of the inner cannula 130. Perimeter 166 of the flat surface 162 is preferably positioned against perimeter 148 of the opening 143. The inner cannula 130 and the stylet 150 are preferably constructed from a polymeric or super-elastic metal.

It will be appreciated that many different and diverse modes and manners for connecting and disconnecting the various hubs 104, 136, 156, and maintaining the desired rotational orientation, all of which are well established devices within the mechanical arts. While the current embodiment uses friction to engage the various hubs 104, 136, 156, a variety of other structures are possible.

Figure 9 illustrates distal end 180 of epidural needle 100 being advanced toward epidural space 52 in accordance with an embodiment of the present disclosure. Inner cannula 130 substantially extends across elongated opening 112 of the outer cannula 102 so that the needle 100 seals against the ligament 72 even though the upper edge 116 of the opening 112 is still located in the muscle tissue 70.

In the configuration illustrated in Figure 9, the surgeon then removes the stylet 150 so that lumen 140 is fluidly coupled with the inner hub 142. The upper edge 141 of the opening 143 is preferably located a distance 147 of about 3 mm to about 4 mm from the distal end 110 of the outer cannula 102. Consequently, the lumen 140 is in fluid communication with the epidural space 52 well before the distal end 110 of the outer cannula 102 reaches the dura 62. Most of the elongated opening 112 of the outer cannula 102 is still substantially sealed by the inner cannula 130.

A source of pressurized air is delivered through the lumen 140 as the surgeon continues to advance the needle 100 toward the epidural space 52. If the distal end 180 of the needle 100 is still positioned within the interspinous ligament 72, the pressure of air will remain generally constant. In embodiments where the source of pressurized air is a syringe fluidly coupled to the lumen 140, the surgeon will encounter resistance at the syringe plunger.

Once the lumen 140 is in fluid communication with the epidural space 52, however, the source of pressurized air will flow into the epidural space 52. Plunger resistance will drop if the surgeon is using a syringe to provide the pressure.

Locating the lumen 140 close to the distal end 110 of the outer cannula 102 permits the present epidural needle 100 to operate in much smaller epidural spaces 182, with reduced risk of damage to the dura 62 or the spinal cord 84. The inner cannula 130 permits the present epidural needle 100 to have an elongated opening 112 suitable for passing a therapy delivery element 14, while performing the loss of resistance technique using only requiring the distal most 110 of the outer cannula 102.

Once the distal end 180 is located in the epidural space 52, the surgeon removes the inner cannula 130, exposing the elongated opening 112 of the outer cannula 102 (see Figure 6B). The therapy delivery element 114 can now be inserted into the epidural space 52, as generally illustrated in Figure 4.

The present epidural needle 100 is suitable to enter the epidural space 52 at any vertebral level, for example, the posterior cervical or thoracic level, or alternatively midline or paramedian at the level of the foramen magnum. Fluoroscopy may be used at any stage of the implantation procedure to ascertain the anatomic position of any particular device or instrumentation. Sterile saline or myelographic dye may be used to dilate epidural space 52 to facilitate passage of therapy delivery elements 14. The present epidural needle 100 may be use along, or in combination with an introducer.

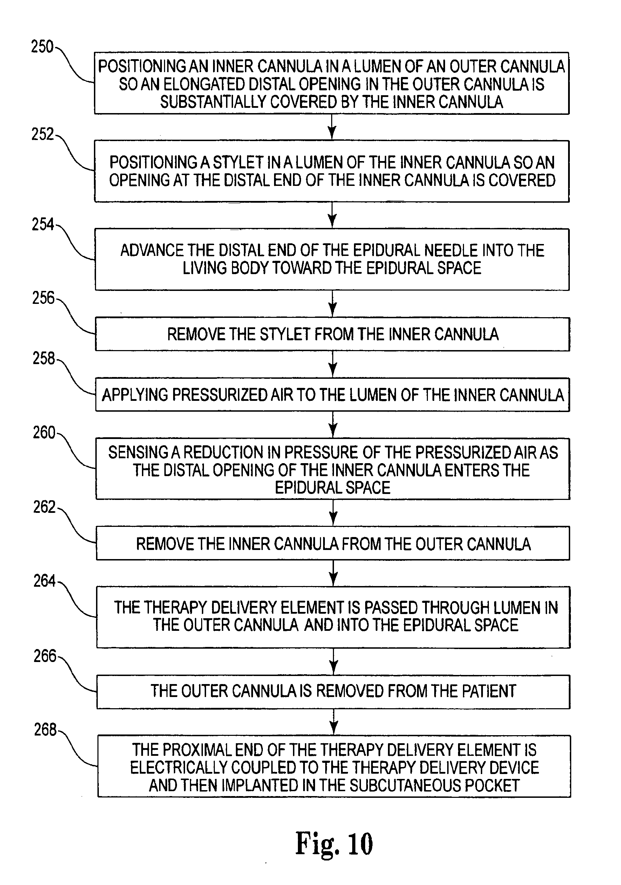

Figure 10 is a flow diagram of a method of implanting a neurostimulation system within a living body using an epidural needle in accordance with an embodiment of the present disclosure. Inner cannula is positioned in lumen of outer cannula so the elongated distal opening is substantially covered by the inner cannula (250). Stylet is positioned in lumen of inner cannula (252). Distal end of epidural needle is advanced through into the living body toward the epidural space (254). The stylet is removed from the inner cannula (256). A source of pressurized air is delivered through the lumen of the inner cannula (258). The surgeon senses changes in pressure of the pressurized air as the distal opening in the inner cannula enters the epidural space (260). Once a drops in pressure is detected the lumen of the inner cannula is in fluid communication with the epidural space. The inner cannula is removed from the outer cannula, exposing the lumen of the outer cannula (262). Distal end of a therapy delivery element is advanced through the lumen of the outer cannula and into the epidural space to a target location within the living body (264). The outer cannula is removed from the patient while leaving the therapy delivery element in the epidural space (266) Proximal ends of the therapy delivery elements are electrically coupled to an implantable pulse generator, either directly or through a lead extension (268).

Where a range of values is provided, it is understood that each intervening value, to the tenth of the unit of the lower limit unless the context clearly dictates otherwise, between the upper and lower limit of that range and any other stated or intervening value in that stated range is encompassed within this disclosure. The upper and lower limits of these smaller ranges which may independently be included in the smaller ranges is also encompassed within the disclosure, subject to any specifically excluded limit in the stated range. Where the stated range includes one or both of the limits, ranges excluding either both of those included limits are also included in the disclosure.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the various methods and materials are now described.

The publications discussed herein are provided solely for their disclosure prior to the filing date of the present application. Nothing herein is to be construed as an admission that the present disclosure is not entitled to antedate such publication by virtue of prior invention. Further, the dates of publication provided may be different from the actual publication dates which may need to be independently confirmed.

Other embodiments are possible. Although the description above contains much specificity, these should not be construed as limiting the scope of the disclosure, but as merely providing illustrations of some of the presently preferred embodiments. It is also contemplated that various combinations or sub-combinations of the specific features and aspects of the embodiments may be made and still fall within the scope of this disclosure. It should be understood that various features and aspects of the disclosed embodiments can be combined with or substituted for one another in order to form varying modes disclosed. Thus, it is intended that the scope of at least some of the present disclosure should not be limited by the particular disclosed embodiments described above.

Thus the scope of this disclosure should be determined by the appended claims. Therefore, it will be appreciated that the scope of the present disclosure fully encompasses other embodiments which may become obvious to those skilled in the art, and that the scope of the present disclosure is accordingly to be limited by nothing other than the appended claims, in which reference to an element in the singular is not intended to mean "one and only one" unless explicitly so stated, but rather "one or more." Moreover, it is not necessary for a device or method to address each and every problem sought to be solved by the present disclosure, for it to be encompassed by the present claims. Furthermore, no element, component, or method step in the present disclosure is intended to be dedicated to the public regardless of whether the element, component, or method step is explicitly recited in the claims.

## Claims

1. An epidural needle for implanting therapy delivery elements in an epidural space, the epidural needle comprising:
at least one outer cannula (102) comprising an outer hub (104) at a proximal end, an elongated opening (112) at a curved distal end, and a lumen (114) extending between the outer hub and the elongated opening, the lumen of the outer cannula sized to permit passage of the therapy delivery elements;
at least one inner cannula (130) including an inner hub (136) at a proximal end, an opening (143) at a distal end, and a lumen (140) extending between the inner hub and the opening, the inner cannula comprising an outer diameter sized to slide freely within the lumen of the outer cannula, the inner and outer hubs including engagement features that maintain the outer cannula in a fixed rotational orientation relative to the inner cannula so that the inner cannula (130) extends substantially across the elongated opening (112) when inserted into the lumen of the outer cannula; and
at least one stylet (150) comprising a stylet hub (156), a distal end, and an outer diameter sized to slide freely within the lumen of the inner cannula, the distal end of the stylet extending substantially across the opening of the inner cannula.

2. The epidural needle according to claim 1 wherein the outer cannula (102), the inner cannula (130) and the stylet (150) comprise a malleable material.

3. The epidural needle according to claim 1 or 2 comprising engagement features on the stylet hub (156) and the inner hub (136) that maintain the inner cannula in a fixed rotational orientation relative to the stylet when the stylet is inserted in the lumen of the inner cannula.

4. The epidural needle according to any one of the preceding claims wherein the elongated opening (112) in the outer cannula (102) comprises an angled distal end with a perimeter edge extending into a portion of a sidewall of the outer cannula.

5. The epidural needle according to claim 4 wherein a perimeter edge of the opening (143) in the inner cannula (130) substantially extends along perimeter edge of the angled distal end of the outer cannula.

6. The epidural needle according to any one of the preceding claims wherein the elongated opening (112) has a length of about 8 mm to about 12 mm and the opening (143) at the distal end of the inner cannula has a length of about 2 mm to about 3 mm.

7. The epidural needle according to any one of the preceding claims wherein an upper edge of opening (143) of the inner cannula is located within about 3 mm to about 4 mm from the distal end of the outer cannula.

8. The epidural needle according to any one of the preceding claims wherein at least one of the inner cannula (130) and the stylet (150) comprise a polymeric material and a super elastic metal.

9. A neurostimulation system comprising:
an implantable pulse generator (12);
a therapy delivery element (14) comprising a proximal end adapted to electrically couple with the implantable pulse generator and a distal end with a plurality of electrodes electrically coupled to the implantable pulse generator; and
the epidural needle according to claim 1.

## Patentansprüche

1. Epiduralnadel zum Implantieren von Therapieabgabeelementen in einen Epiduralraum, wobei die Epiduralnadel umfasst:
mindestens eine äußere Kanüle (102), umfassend einen äußeren Ansatz (104) an einem proximalen Ende, eine längliche Öffnung (112) an einem gebogenen distalen Ende, und ein Lumen (114), das sich zwischen dem äußeren Ansatz und der länglichen Öffnung erstreckt; wobei das Lumen der äußeren Kanüle so dimensioniert ist, dass es die Passage der Therapieabgabeelemente zulässt;
mindestens eine innere Kanüle (130), beinhaltend einen inneren Ansatz (136) an einem proximalen Ende, eine Öffnung (143) an einem distalen Ende, und ein Lumen (140), das sich zwischen dem inneren Ansatz und der Öffnung erstreckt, wobei die innere Kanüle einen Außendurchmesser aufweist, der so dimensioniert ist, dass sie in dem Lumen der äußeren Kanüle frei verschiebbar ist, wobei der innere und äußere Ansatz Eingriffseigenschaften beinhalten, welche die äußere Kanüle in Bezug zu der inneren Kanüle in einer feststehenden Drehorientierung halten, sodass die innere Kanüle (130), wenn sie in das Lumen der äußeren Kanüle eingebracht ist, sich im Wesentlichen über die längliche Öffnung (112) erstreckt; und
mindestens einen Mandrin (150), umfassend einen Mandrinansatz (156), ein distales Ende, und einen Außendurchmesser, der so dimensioniert ist, dass er in dem Lumen der inneren Kanüle frei verschiebbar ist, wobei das distale Ende des Mandrins sich im Wesentlichen über die Öffnung der inneren Kanüle erstreckt.

2. Epiduralnadel nach Anspruch 1, wobei die äußere Kanüle (102), die innere Kanüle (130) und der Mandrin (150) ein plastisches Material umfassen.

3. Epiduralnadel nach Anspruch 1 oder 2, umfassend Eingriffseigenschaften an dem Mandrinansatz (156) und dem inneren Ansatz (136), welche die innere Kanüle in einer feststehenden Drehorientierung in Bezug zu dem Mandrin halten, wenn der Mandrin in das Lumen der inneren Kanüle eingebracht ist.

4. Epiduralnadel nach einem der vorgenannten Ansprüche, wobei die längliche Öffnung (112) in der äußeren Kanüle (102) ein angewinkeltes distales Ende mit einem Umfangsrand umfasst, der sich in einen Teil einer Seitenwand der äußeren Kanüle erstreckt.

5. Epiduralnadel nach Anspruch 4, wobei ein Umfangsrand der Öffnung (143) in der inneren Kanüle (130) sich im Wesentlichen entlang dem Umfangsrand des angewinkelten distalen Endes der äußeren Kanüle erstreckt.

6. Epiduralnadel nach einem der vorgenannten Ansprüche, wobei die längliche Öffnung (112) eine Länge von etwa 8 mm bis etwa 12 mm aufweist und die Öffnung (143) am distalen Ende der inneren Kanüle eine Länge von etwa 2 mm bis etwa 3 mm aufweist.

7. Epiduralnadel nach einem der vorgenannten Ansprüche, wobei ein oberer Rand der Öffnung (143) der inneren Kanüle innerhalb von etwa 3 mm bis etwa 4 mm vom distalen Ende der äußeren Kanüle angeordnet ist.

8. Epiduralnadel nach einem der vorgenannten Ansprüche, wobei mindestens eines der inneren Kanüle (130) und des Mandrins (150) ein Polymermaterial und ein superelastisches Metall umfassen.

9. Neurostimulationssystem, umfassend:
einen implantierbaren Impulsgenerator (12);
ein Therapieabgabeelement (14), umfassend ein proximales Ende, das zur elektrischen Koppelung mit dem implantierbaren Impulsgenerator eingerichtet ist, und ein distales Ende mit einer Vielzahl von Elektroden, die elektrisch an den implantierbaren Impulsgenerator gekoppelt sind; und
die Epiduralnadel nach Anspruch 1.

## Revendications

1. Aiguille épidurale pour l'implantation d'éléments de distribution thérapeutique dans un espace épidural, l'aiguille épidurale comprenant :
au moins une canule externe (102) comprenant un raccord externe (104) à une extrémité proximale, une ouverture allongée (112) à une extrémité distale courbe et une lumière (114) s'étendant entre le raccord externe et l'ouverture allongée, la lumière de la canule externe étant dimensionnée pour permettre le passage d'éléments de distribution thérapeutique ;
au moins une canule interne (130) englobant un raccord interne (136) à une extrémité proximale, une ouverture (143) à une extrémité distale, et une lumière (140) s'étendant entre le raccord interne et l'ouverture, la canule interne comprenant un diamètre externe dimensionné pour coulisser librement au sein de la lumière de la canule externe, les raccords interne et externe englobant des caractéristiques de mise en contact qui maintiennent la canule externe dans une orientation fixe en rotation par rapport à la canule interne, si bien que la canule interne (130) s'étend essentiellement à travers la l'ouverture allongée (112) après son insertion dans la lumière de la canule externe ; et
au moins un stylet (150) comprenant un raccord de stylet (156), une extrémité distale, et un diamètre externe dimensionné pour coulisser librement au sein de la lumière de la canule interne, l'extrémité distale du stylet s'étendant essentiellement à travers l'ouverture de la canule interne.

2. Aiguille épidurale selon la revendication 1, dans laquelle la canule externe (102), la canule interne (130) et le stylet (150) comprennent une matière malléable.

3. Aiguille épidurale selon la revendication 1 ou 2, comprenant des caractéristiques de mise en contact sur le raccord de stylet (156) et le raccord interne (136) qui maintiennent la canule interne dans une orientation fixe en rotation par rapport au stylet, lorsque le stylet est inséré dans la lumière de la canule interne.

4. Aiguille épidurale selon l'une quelconque des revendications précédentes, dans laquelle l'ouverture allongée (112) dans la canule externe (102) comprend une extrémité distale angulaire dont le bord périphérique s'étend dans une portion d'une paroi latérale de la canule externe.

5. Aiguille épidurale selon la revendication 4, dans laquelle un bord périphérique de l'ouverture (143) dans la canule interne (130) s'étend essentiellement le long du bord périphérique de l'extrémité distale angulaire de la canule externe.

6. Aiguille épidurale selon l'une quelconque des revendications précédentes, dans laquelle l'ouverture allongée (112) possède une longueur d'environ 8 mm à environ 12 mm et l'ouverture (143) à l'extrémité distale de la canule interne possède une longueur d'environ 2 mm à environ 3 mm.

7. Aiguille épidurale selon l'une quelconque des revendications précédentes, dans laquelle un bord supérieur de l'ouverture (143) de la canule interne est situé à une distance en-deçà d'environ 3 mm à environ 4 mm par rapport à l'extrémité distale de la canule externe.

8. Aiguille épidurale selon l'une quelconque des revendications précédentes, dans laquelle au moins un membre choisi parmi le groupe comprenant la canule interne (130) et le stylet (150) comprend une matière polymère et un métal superélastique.

9. Système de neurostimulation comprenant :
un générateur d'impulsions implantable (12) ;
un élément de distribution thérapeutique (14) comprenant une extrémité proximale conçue pour s'accoupler par voie électrique au générateur d'impulsions implantable et une extrémité distale comprenant plusieurs électrodes couplées par voie électrique au générateur d'impulsions implantable ; et
l'aiguille épidurale selon la revendication 1.
